# EUROPEAN PATENT SPECIFICATION

(11) **EP 1 995 326 B1**
(45) Date of publication and mention of the grant of the patent: **16.10.2013**
(21) Application number: 06840372.4
(22) Date of filing: 26.12.2006
(51) Int. Cl.: C12Q 1/68, C12N 15/09

(54) **TWO STAGE METHODS FOR EXAMINING NUCLEIC ACID BY USING ONE AND THE SAME SAMPLE**
ZWEISTUFIGE VERFAHREN ZUR UNTERSUCHUNG VON NUKLEINSÄURE DURCH VERWENDUNG EIN UND DERSELBEN PROBE
PROCEDES EN DEUX ETAPES POUR EXAMINER UN ACIDE NUCLEIQUE EN UTILISANT UN SEUL ECHANTILLON

(30) Priority: 16.03.2006 JP 2006072581
(43) Date of publication of application: 26.11.2008
(73) Proprietor: Japan Software Management Co., Ltd., Yokohama-shi Kanagawa 221-0056 (JP); Igarashi, Takao, Yokohama-shi Kanagawa 244-0813 (JP); Nasu, Hisanori, Yokohama-shi Kanagawa 245-0018 (JP)
(72) Inventor: NASU, Hisanori, Yokohama-shi, Kanagawa 2450018 (JP); TSUJIMOTO, Atsumi, Yokohama-shi, Kanagawa 221-0056 (JP); KOBAYASHI, Takanori, Yokohama-shi, Kanagawa 231-0802 (JP)
(74) Representative: Woods, Geoffrey Corlett
(86) International application number: PCT/JP2006/325982
(87) International publication number: WO 2007/108193

(56) References cited:
- EP-A1- 1 582 599
- JP-A- 02 299 600
- JP-A- 2002 539 769
- JP-A- 2004 057 207
- US-A1- 2004 229 211
- TRAMA J.P. ET AL.: 'Detection and identification of Candida species associated with Candida vaginitis by real-time PCR and pyrosequencing' MOL. CELL PROBES vol. 19, 2005, pages 145 - 152, XP004725277
- YANG G. ET AL.: 'Flow cytometric immunodetection of human immunodeficiency virus type 1 proviral DNA by heminested PCR and digoxigenin-labeled probes' CLIN. DIAGN. LAB. IMMUNOL. vol. 1, no. 1, 1994, pages 26 - 31, XP009005413
- BAEZ L.A. ET AL.: 'Chemiluminescent enzyme immunoassay for detection of PCR-amplified enterotoxin A from Clostridium perfringens' INT. J. FOOD MICROBIOL. vol. 32, 1996, pages 145 - 158, XP003017834
- POURMAND N. ET AL.: 'Multiplex pyrosequencing' NUCLEIC ACIDS RES. vol. 30, no. 7, 2002, pages E31-1 - E31-5, XP002329580

## Description

### TECHNICAL FIELD

The present invention relates to a method of testing whether a sequence of interest is contained in a sample nucleic acid. The nucleotide sequences of DNA possessed by an organism differ from each other by individual, species, habitat, and so forth. Therefore, by using the method of the present invention, it is possible to discriminate the individual, species, habitat, etc. of an organism. Recently, putting false labels on foods or mixing of genetically modified products have become issues. The method of the present invention is also applicable to judgment of such false labels or mixing.

### BACKGROUND ART

Generally, tuna may be classified into bluefin tuna (*Thunnus thynnus*) grown in the Pacific Ocean, bluefin tuna grown in the Atlantic Ocean, Southern bluefin tuna (*Thunnus maccoyii*), big-eyed tuna (*Thunnus obesus*), Humphead thryssa (*Thunnus albacares*), albacore (*Thunnus alalunga*) and so forth. Conventionally, a technique called polymerase chain reaction-restriction fragment length polymorphism (PCR-RFLP; see, for example, Non-Patent Document No. 1) has been used for discriminating types of tuna (see, for example, Non-Patent Document No. 2). This technique comprises amplifying a sample DNA by PCR (see, for example, Non-Patent Document No. 3); digesting the DNA at sites of the nucleotide sequence occurring specifically in a species, lineage or individual; and making judgment. This method discriminate the species, lineage or individual of interest from the region of DNA amplified, the type of the restriction enzyme for digesting the DNA, the number of DNA fragments resulting from the digestion and the length of each DNA fragment. For example, when the tuna of interest is bluefin tuna grown in the Pacific Ocean, the ATCO region of mitochondrial DNA is selected as a sample DNA. When this sample DNA is digested with a restriction enzyme AluI after PCR amplification, five DNA fragments are generated of which the base lengths are 432, 295, 81, 66 and 41. When this sample DNA is digested with a restriction enzyme MseI, eight DNA fragments are generated of which the base lengths are 255, 254, 194, 115, 41, 32, 15 and 9 are generated. When this sample DNA is digested with a restriction enzyme Tsp509I, six DNA fragments are generated of which the base lengths are 417, 217, 186, 72, 15 and 8 (see, for example, Non-Patent Document No. 2).

Similar techniques are also used in testing and judging genetically modified products.

Patent Document No. 1 describes multiplex real-time PCR testing of genetic materials by using two or more primers with corresponding specific probe sets using the same reporter gene. Non-Patent Document No. 4 describes methods that combine real-time PCR and pyrosequencing to amplify sequence ribosomal DNA from Candida albicans. Patent Document No. 2 describes the sorting and purification of microbeads having bound nucleic acids.

[Non-Patent Document No. 1] G R. Deng, "A sensitive non-radioactive PCR-RFLP analysis for detecting point mutations at 12th codon of oncogene c-Ha-ras in DNAs of gastric cancer", Nucleic Acids Res. 1988 July 11; 16(13): 6231.

[Non-Patent Document No. 2] IAA Center for Food Quality, Labeling and Consumer Services/Fisheries Research Agency, "Technical Information: Manual for Typing Fishes of Thunnus".

[Non-Patent Document No. 3] Mullis, K. and five others, "Specific enzymatic amplification of DNA in vitro: The polymerase chain reaction", Cold Spring Harb Symp Quant Biol. 1986; 51 Pt 1:263-73

[Non-Patent Document No. 4] Trama et al, Mol Cell Probes (2005) 19:145-152.

[Patent Document No. 1] US 2004/229211.

[Patent Document No. 2] EP A 1 582 599.

### DISCLOSURE OF THE INVENTION

### PROBLEM FOR SOLUTION BY THE INVENTION

The above-described technique of PCR-RFLP is a discrimination method using differences in the number of DNA fragments resulting from the digestion with a restriction enzyme and in the lengths of the individual DNA fragments. Specifically, when all the digested DNA fragments have been separated by electrophoresis, the separated individual DNA fragments appear as bands. At this time, the number of bands appearing is the number of DNA fragments; and the distance of migration of each band is the length of the relevant DNA fragment. Electrophoresis is a method of separation utilizing the effect that shorter DNA fragment migrate rapidly while longer DNA fragment migrate slowly. The lengths of DNA fragments can be determined from their distances of migration.

However, electrophoresis is not capable of sufficiently separating DNA fragments when a plurality of digested DNA fragments have similar lengths. Even when two DNA fragments are present, they appear as one band. (For example, in a fluorescently stained image of electrophoresis gel as shown in Non-Patent Document No. 2, two DNA fragments of 255 base length and 254 base length are only detected as one broad band when they were electrophoresed a distance of about 4 cm on 3% (w/v) agarose gel.) Therefore, when PCR-RFLP is performed under conditions which generate DNA fragments of similar lengths, the number of DNA fragments may be judged erroneously. Thus, the technique of PCR-RFLP is not highly reliable and unsuitable for important tests.

Further, the phenomenon that DNA fragments of similar lengths can not be separated is not a problem peculiar to PCR-RFLP, but it is a common problem encountered by every testing technique using electrophoresis. Therefore, not only PCR-RFLP but other testing techniques which determine the number of DNA fragments by electrophoresis such as qualitative PCR [for example, Japanese Unexamined Patent Publication No. 2003-009866 titled "Animal Species Discrimination Method with SINE Technique"; Notice regarding Testing Method for Foods Produced by Recombinant DNA Technique (SHOKU-HATSU No. 110 issued on March 27, 2001) (attachment: "Testing Method for Foods Produced by Recombinant DNA Technique" , this document is available for download from the website of the Ministry of Health, Labor and Welfare http://www.mhlw.go.jp/topics/idenshi/kensa/050517a.html)] are not highly reliable and thus unsuitable for important tests.

On the other hand, the most reliable testing technique is testing by reading the nucleotide sequence of sample DNA (for example, see Sambrook, J. & Russell, D.W., Chapter 12 DNA Sequencing in Molecular Cloning: A laboratory manual, 3rd Ed. 2001, pp. 12.1-12.120). However, this method requires high cost and can be performed only in special laboratories provided with special equipment and skilled technicians. Thus, this method is not suitable for ordinary, frequent tests.

The present invention has been made in view of these problems conventional tourniquets have. It is an object of the present invention to provide a more efficient testing method using DNA.

### MEANS TO SOLVE THE PROBLEM

As a result of extensive and intensive researches toward the solution of the above-described problem, the present inventors have found that it is possible to perform a highly reliable test with low cost by dividing the test into two states of a simple test and a close examination when testing whether or not a sequence of interest is contained in a sample nucleic acid. The present invention has been achieved based on this finding.

The present invention provides the following (1) to (12).
(1) A method of testing whether a sequence of interest is contained in a sample nucleic acid or not, comprising performing a simple test on the sample nucleic acid wherein the simple test is a quantitative test on the sample nucleic acid, and subjecting the same sample nucleic
   acid to a close examination when it has been judged that the sequence of interest is contained therein in more than a specific amount, wherein the close examination is an examination performed by reading the nucleotide sequence of the sample nucleic acid, wherein said method is carried out using a device in which
      (a) a DNA extraction unit (21), detector unit (22) and DNA storage unit (23) are engraved on a flat board; and
      (b) said DNA extraction unit (21) is connected to said detector unit (22) by a thin groove and said DNA extraction unit (21) is connected to said DNA storage unit (23) by a thin groove; and
      (c) a lid (24), which is a flat board, is placed on the device, wherein said lid has a hole (25) located above the DNA extraction unit (21) through which samples can be fed into said DNA extraction unit;
         wherein said method comprises
         feeding a sample through the hole (25) into the DNA extraction unit (21), extracting DNA from the sample in DNA extraction unit (21),
         dividing the extracted DNA in said DNA extraction unit (21) into two aliquots,
         delivering the first aliquot to the detector unit (22) and simultaneously delivering the second aliquot to the DNA storage unit (23), and
         carrying out the simple test on said first aliquot in the detector unit (22)
         wherein the two aliquots are both stored on the same device for performing the simple test and the close examination.
(2) The method according to (1) above, wherein the sequence of interest is a sequence occurring specifically in a species of an organism.
(3) The method according to (1) above, wherein the sequence of interest is a sequence occurring specifically in a genetically modified organism.
(4) The method according to (1) above, wherein a plurality of sequences of interest can be tested simultaneously in one simple test.
(5) The method according to any one of (1) to (3) above, wherein said close examination comprises opening said lid (24), recovering the DNA retained in the DNA storage unit (23) and/or the detector unit (22) and carrying out a close examination on said DNA.

### EFFECT OF THE INVENTION

The present invention produces the effects as described below.

By performing a two-stage test as shown in the method of (1) above, efficient and highly reliable testing becomes possible. That is, usually, a simple test of the first stage which does not require special equipment or skilled technicians and yields results with low cost and in a short period of time is performed; and when a problem has been found, then, a close examination of the second stage is performed.

Further, according to a method in which an examination by reading the nucleotide sequence of sample nucleic acid is employed as a close examination of the second stage as described in the method of (1) above, highly reliable testing can be secured regardless of what method has been used as a simple test in the first stage, because reading of the nucleotide sequence of nucleic acid is the most reliable analysis/test method for the primary structure of nucleic acid.

Further, according to a method in which a sample nucleic acid is tagged in advance and the sample nucleic acid is recovered after a simple test using the tag, it is possible to efficiently recover the sample nucleic acid from non-tagged substances.

Further, according to a method in which a sample nucleic acid is recovered after a simple test using a physicochemical difference between the sample nucleic acid and other substance, it is possible to recover the sample nucleic acid without addition of a tag thereto.

Further, according to a testing method in which a sample nucleic acid is divided into two aliquots in advance, and one is used in a simple test and the other in a close examination as described in the method of (1) above, it is possible to secure the sample for the close examination even when the simple test of the first stage disrupts the sample nucleic acid. Further, it is also possible to reduce mistakes such as confusion of test samples by retaining the divided aliquots on the same device.

Further, according to a method in which a plurality of sequences of interest can be tested in one simple test as described in the method of (3) above, it is possible to test a plurality of sequences of interest at one time.

### BEST MODE FOR CARRYING OUT THE INVENTION

Hereinbelow, the present invention will be described in detail.

The testing method of the invention is a method of testing whether a sequence of interest is contained in a sample nucleic acid in more than a specific amount or not, comprising performing a simple quantitative test on the sample nucleic acid and performing a close examination on the same sample nucleic acid when it has been judged that the sequence of interest is contained in more than the specific amount. By thus dividing a test into two stages and performing a close examination only when a problem is found, it is possible to provide highly reliable testing with optimum cost. This technique is a testing method suitable for situations where problems occur only in a small number of cases.

Sequences of interest to be tested are not particularly limited. Since the testing methods of the present invention are used in judging the species of organisms or judging genetically modified organisms, sequences occurring specifically in the species, habitats or individuals of organisms or sequences occurring specifically in genetically modified organisms may be used as sequences of interest to be tested. Information about such specific sequences is widely known. For example, information about sequences specific to species or habitats of tuna fishes is disclosed in Non-Patent Document No. 3 (this document is available for download from the website of IAA Center for Food Quality, Labeling and Consumer Servicces (<URL:http: //www.cfqlcs.go.jp/technical_information/hinpyou/pdf/maguro_manual.pdf>). Information about species specific sequences of Alaskan king crab (*Paralithodes camtschatica*) and blue king crab (*Paralithodes platypus*) is disclosed in H. Uyama, M. Ikeda and N. Taniguchi, "Discrimination of Paralithodes camtschatica and Paralithodes platypus by PCR-RFLP of mtDNA", Fish Genetics and Breeding Science, 2005, 34:111. Information about species specific sequences of Hexagrammidae fishes is disclosed in S. Yanagimoto and Y. Hamatsu, "Discrimination of 7 Species of Hexagrammidae Fishes Grown in Japan by PCR-RFLP Analysis of mtDNA", Journal of Japan Society of Fisheries Science, 2003, 69 (5): 726. Information about species specific sequences of sea bream is disclosed in "Technical Information: Species Discrimination Manual for *Pagrus major*, *Evynnis japonica* and *Dentex tumifrons*" (this document is available for download from the website of IAA Center for Food Quality, Labeling and Consumer Servicces, <URL:http: //www.cfqlcs.go.jp/technical_information/hinpyou/pdf/madai_manual.pdf>). Information about species specific sequences of Bass (*Leteolabrax japonicus*) and Nile perch (*Lates niloticus*) is disclosed in "Technical Information: Species Discrimination Manual for *Leteolabrax japonicus*, *Leteolabrax* sp. and *Lates niloticus*" (this document is available for download from the website of IAA Center for Food Quality, Labeling and Consumer Servicces, <URL:http://www.cfqlcs.gojp/technical_information/hinpyou/pdf/suzuki_manual.pdf>). Information about species specific sequences of fishes belonging to the genus *Scomber* grown in Japan is disclosed in Japanese Unexamined Patent Publication No. 2002-345498 titled "Species Discrimination Method for Japanese *Scomber* Fishes". Information about sequences specific to genetically modified organisms that were approved of 1st type use and 2nd type use under Cartagena Protocol is obtainable by consulting LMO related information "Search System for Genetically Modified Organisms Approved under Cartagena Protocol" (this document is available for download from the website of Japan Biosafety Clearing-House (J-BCH); <URL:https://ch.biodic.go.jp/bch/OpenSearch.do>).

Information about specific sequences other than those described above is also readily available for those skilled in the art. It should be noted here that sequences to be tested include not only DNA sequences but also RNA sequences.

The method of simple test is not particularly limited. When a sample nucleic acid is divided into two aliquots and one is used in a simple test and the other in a close examination, either a method which disrupts the sample nucleic acid or a method which does not disrupt the sample nucleic acid may be used as a simple test. Specific examples of methods which disrupt a sample nucleic acid include, but are not limited to, RFLP method, PCR-RFLP method, AFLP method, Southern blotting, chemical cleavage of mismatch (CCM) and methods using nuclease. Specific examples of methods which do not disrupt a sample nucleic acid include, but are not limited to, methods using hybridization in solution, real time PCR, primer extension method, sequencing method, random amplified polymorphic DNA method, TaqMan assay and Invader assay.

The method of recovering nucleic acid is not particularly limited. Preferably, a method used in which a sample nucleic acid is tagged in advance and the sample nucleic acid is recovered using the tag after a simple test. Alternatively, a method may be used in which a sample nucleic acid is recovered after a simple test using a physicochemical difference between the sample nucleic acid and other substance. Specific examples of recovering methods using a physicochemical difference include use of columns, electrophoresis and extraction with organic solvents.

The device for a simple test of the first stage may be made into a microchip using the microfluidics technology or the like. This enables automatic testing with a small-sized instrument. Further, a sample nucleic acid to be recovered after the simple test can be retained sealed in the microchip and delivered to the place of a close examination of the second stage easily and safely. In the close examination, the sample nucleic acid can be recovered from this microchip and used.

The method of close examination is a testing method of reading the nucleotide sequence of a sample nucleic acid is used.

Hereinbelow, preferred embodiments of the testing method of the invention are described with reference to Figures.

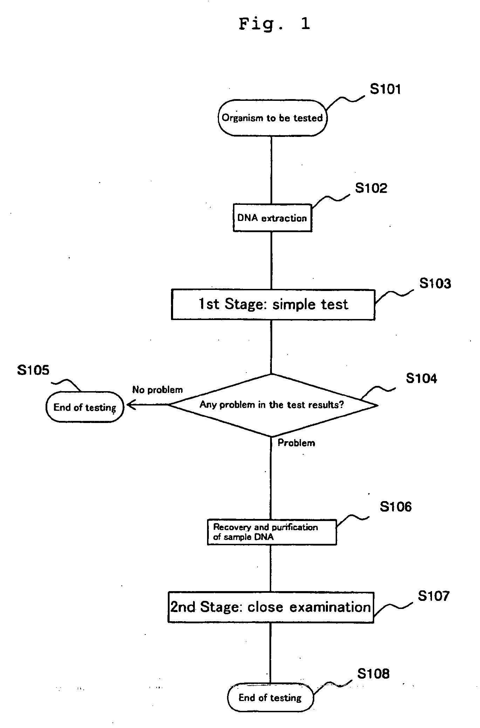

Fig. 1 illustrates a flow of a testing method where a simple test which does not disrupt a sample nucleic acid is employed, and the sample nucleic acid used in the simple test is recovered. First, from an organism to be tested (S 101), DNA containing a sample nucleic acid is extracted (S102). With this DNA, a simple test of the first stage is performed (S103), followed by judgment of the results of the simple test (S 104). When no problem is found, the testing is finished (S 105). When a problem is found in the results of the simple test, the sample nucleic acid which was judged to have a problem in the simple test of the first stage is recovered and purified (S106). With this recovered/purified sample, a close examination of the second stage is performed (S107). The results are judged and the testing is finished (S108).

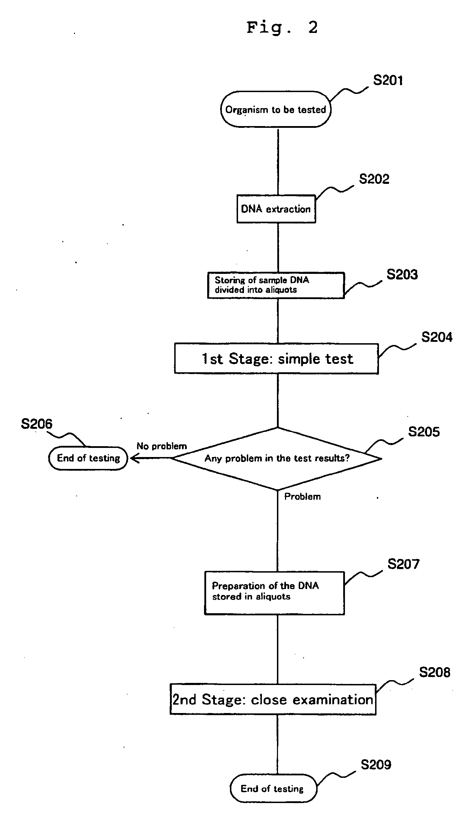

Fig. 2 illustrates a flow of the testing method of the invention where a simple test which disrupts a sample nucleic acid is employed. First, from an organism to be tested (S201), DNA containing a sample nucleic acid is extracted (S202). Then, the sample DNA is divided into aliquots and stored on the same device in advance (S203), and a simple test of the first stage is performed using one aliquot thereof (S204). The results of the simple test are judged (S205). When no problem is found, the testing is finished (S206). When a problem is found in the results of the simple test, an aliquot of the sample nucleic acid stored for the second stage is used (S207) to perform a close examination of the second stage (S208). The results are judged and the testing is finished (S209).

As described above, according to the preferred embodiments of the present invention, it becomes possible to perform a two-step testing. As a first stage, a simple test which does not require special equipment or skilled technicians and can be done easily and at low cost is performed. Then, when a problem is found in the results, a highly reliable close examination of the second stage is performed using the same sample DNA recycled. Thus, it is possible to provide a testing much more reliable than conventional testing. As a close examination of the second stage, the present invention employs a method of reading the nucleotide sequence of DNA which is currently the most reliable method. By employing this method, it is possible to provide ultimately the most reliable results even when the results of the simple test at the first stage have some problem.

Subsequently, a best mode for carrying out a method where a simple test of the first stage does not disrupt a sample DNA and the sample DNA recovered therefrom is used in a close examination of the second stage will be described below.

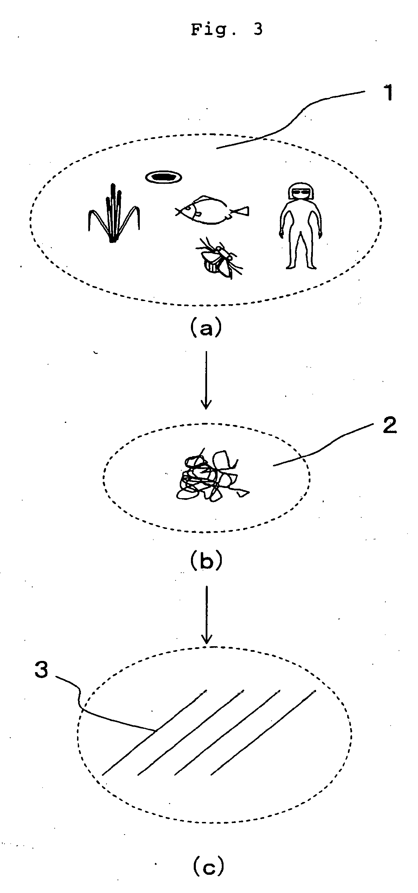

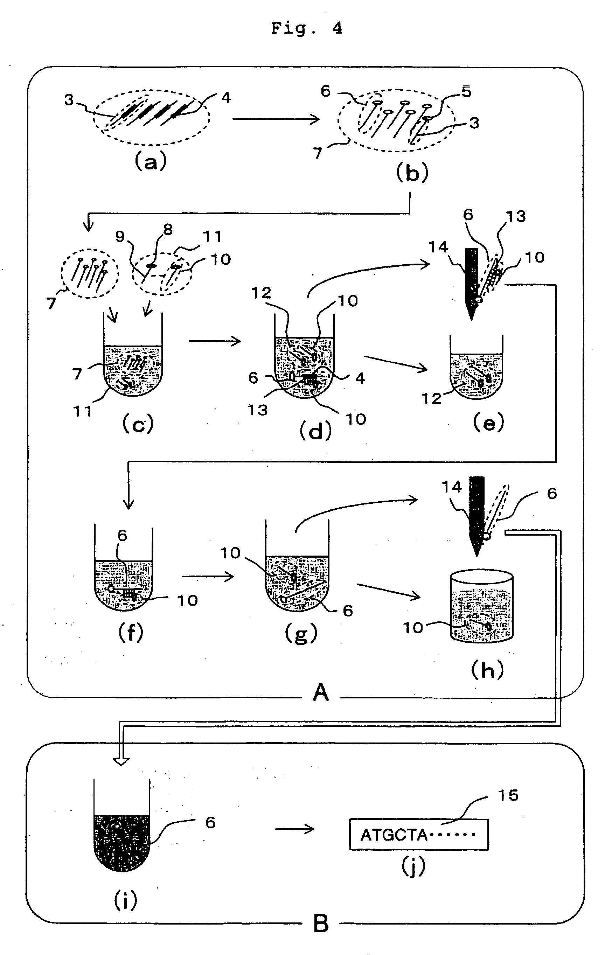

Fig. 3 illustrates a flow of preparation of a sample DNA from an organism to be tested. Fig. 4 illustrates a flow of testing where a sample DNA used in test A of the first stage is recovered and recycled to test B of the second stage. First, from a group of various organisms to be tested as illustrated in (a) in Fig. 3, nucleic acid from an organism to be tested 2 as shown in (b) is extracted by known techniques (see, for example, Sambrook J & Russell DW, Chapter 6 Preparation and analysis of eukaryotic genomic DNA, in Molecular Cloning: A laboratory manual. 3rd Ed. 2001, pp. 6.1-6.64; and Sambrook J & Russell DW, Chapter 7 Extraction, purification, and analysis of mRNA from eukaryotic cells, in Molecular Cloning: A laboratory manual. 3rd Ed. 2001, pp. 7.1-7.94). The organism to be tested may be other than those illustrated in this Figure. The resultant DNA is partially amplified and/or reverse-transcribed, if necessary, to thereby obtain a necessary amount of sample DNA 3 as shown in (c). It is an object of the testing method of the present invention to identify whether or not this sample DNA 3 contains a nucleotide sequence of interest 4 as shown in (a) in Fig. 4. Fig. 4 illustrates a flow of testing where DNA hybridization is used as an example of simple test A of the first stage which does not disrupt the sample DNA; and reading of the DNA nucleotide sequence is used as close examination B of the second stage. At both stages, the same sample DNA 3 is used. Since hybridization does not disrupt sample DNA, is much simpler in reaction system than analysis of the nucleotide sequence of DNA and can be done at low cost, it is a method suitable for a simple test that recovers sample DNA after testing.

First, as shown in (b) in Fig. 4, magnetic beads 5 are attached to one end of sample DNA 3 prior to testing to thereby prepare bead-bound sample DNA 6. A group of bead-bound sample DNA molecules 7 with this structure is used in a two-step testing. By these operations, it is possible to purify and recover this group of molecules alone from contaminants by means of magnetic attraction after test A of the first stage, and to recycle to test B of the second stage.

After preparation of the group of bead-bound sample DNA molecules 7, DNA hybridization (simple test of the first stage) is performed. For this purpose, a fluorescently labeled probe 10 is prepared by attaching a fluorescent dye 8 to an end of a probe DNA 9 that has a complementary nucleotide sequence to nucleotide sequence of interest 4, as shown in (c) in Fig. 4. Then, a group of molecules of this fluorescently labeled probe 11 in solution and the group of bead-bound sample DNA molecules 7 in solution are put in a reaction vessel and mixed therein. It should be noted here that the number of molecules in the group of fluorescently labeled probe 11 should be excessive to the number of molecules in the group of bead-bound sample DNA 7. After heating, reaction vessel is gradually cooled and retained at an appropriate temperature. By these operations, it is possible to hybridize nucleotide sequence of interest 4 in the bead-bound sample DNA 6 to the fluorescently labeled probe 10 in the reaction solution to thereby allow double strand formation 13. By this double strand formation 13, it is possible to indirectly attach the fluorescent dye 8 to nucleotide sequence of interest 4. By detecting the fluorescence emitted by the fluorescent dye, it is possible to detect the presence of nucleotide sequence of interest 4 in the reaction solution.

However, since the group of fluorescently labeled probe 11 is present in the reaction solution in excess to the group of bead-bound sample DNA 7, a group of excessive, unreacted fluorescently labeled probe 12 remains and also emits fluorescence. Therefore, for accurate testing, it is necessary to remove this group of unreacted fluorescently labeled probe 12 and to detect only the fluorescence from the fluorescently labeled probe 10 which has undergone double strand formation. For this purpose, a magnetic pipette 14 may be used to attract and fix the magnetic beads in the reaction solution. By this operation, it becomes possible to recover the bead-bound sample DNA 6 and the fluorescently labeled probe 10 (forming a double strand with nucleotide sequence of interest 4 within sample DNA 6) alone, as shown in (e) in Fig. 4. Therefore, it is possible to recover only the necessary molecules while leaving the group of unreacted fluorescently labeled probe 12 in the reaction vessel. Thereafter, as shown in (f), the bead-bound sample DNA 6 and the fluorescently labeled probe 10 forming a double strand therewith which have been thus recovered are suspended in a fresh solution. By detecting fluorescence in this solution, it is possible to detect the presence of nucleotide sequence of interest 4 in sample DNA 3. When there is any problem in the results of this test, the sample DNA is recovered and subjected to test B of the second stage.

At this stage, the reaction solution after simple test A of the first stage contains the bead-bound sample DNA 6 and the fluorescently labeled probe 10 (forming a double strand with nucleotide sequence of interest 4 within sample DNA 6). Of these, what is necessary for close examination B of the second stage is the bead-bound sample DNA 6 alone. Since the fluorescently labeled probe 10 becomes a contaminant that inhibits close examination B of the second stage, it is necessary to purify and recover the bead-bound sample DNA 6 alone prior to the close examination. For this purpose, after the completion of test A of the first stage, as shown in (g), reaction vessel containing the reaction solution is heated to dissociate hydrogen bonds forming the double strand by heat denaturation, followed by recovery of the bead-bound sample DNA 6 alone from the reaction solution with a magnetic pipette 14 as shown in (h). Thus, it is possible to recover only the necessary molecules while leaving the dissociated fluorescently labeled probe 10 in the reaction vessel.

Subsequently, as shown in (i), the bead-bound sample DNA is suspended in a solution for reading the nucleotide sequence thereof and recycled to close examination B of the second stage, in which the nucleotide sequence thereof is read by known methods (for example, see Sambrook J. et al. mentioned above). Then, the presence of nucleotide sequence 15 corresponding to nucleotide sequence of interest 4 in sample DNA 3 is detected (j).

As described above, it is possible to perform test A of the first stage simply and with low cost; and when there is any problem in the results, then it is possible to perform highly reliable close examination B of the second stage recovering and recycling the sample DNA used in test A of the first stage.

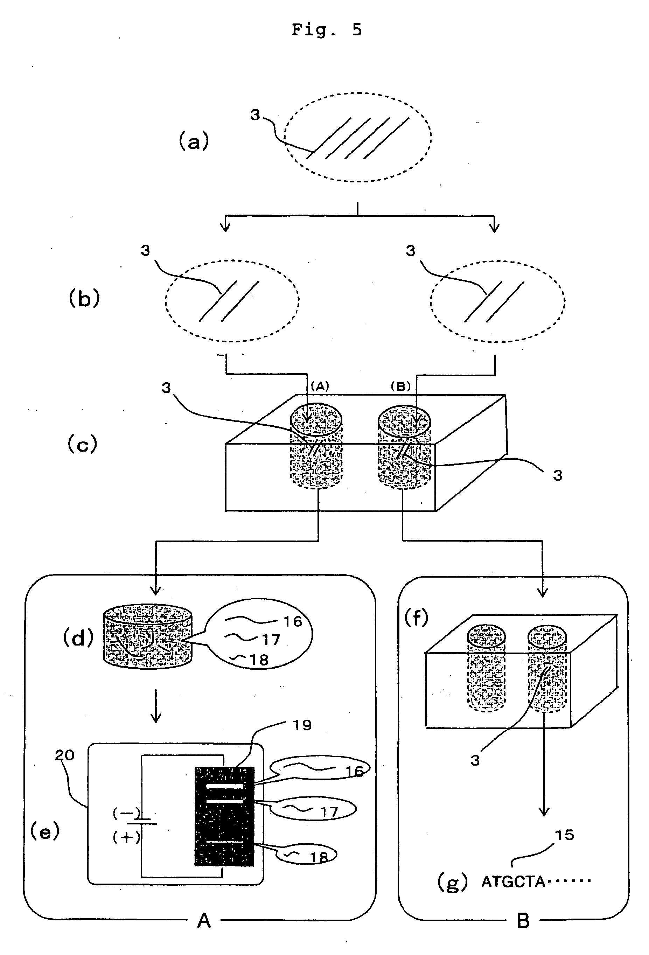

Subsequently, a preferred embodiment for carrying out a method where simple test A of the first stage disrupts a sample DNA will be described below. In this method, the sample DNA are divided into aliquots for test A of the first stage and close examination B of the second stage in advance and stored on the same device. Fig. 5 illustrates a flow of two-step testing using a sample DNA stored in aliquots when PCR-RFLP method involving DNA disruption is used as simple test A of the first stage.

First, a necessary amount of sample DNA 3 is obtained in the same manner as in the above-described case where sample DNA is recycled, according to Fig. 3 which illustrates a flow of preparation of sample DNA from an organism to be tested. This DNA is used as a starting material for testing (Fig. 5, (a)). As shown in (b), this DNA is divided into two aliquots, one for simple test A of the first stage and the other for close examination B of the second stage. These aliquots are stored at place (A) and place (B), respectively, on the same device as shown in (c). A restriction enzyme is added to the aliquot of sample DNA 3 stored at place (A) for simple test A of the first stage. As a result, as shown in (d), sample DNA 3 is digested into a long DNA fragment 16, a medium-length DNA fragment 17, and a short DNA fragment 18. Subsequently, as shown in (e), a solution containing these DNA fragments is applied to an electrophoresis gel 19 to perform electrophoresis using an electrophoresis apparatus 20. Thus, three DNA fragments are separated on the gel, which is stained with a dye capable of staining nucleic acid. When an electrophoretic pattern as expected has been obtained, it is judged that nucleotide sequence of interest 4 is contained in sample DNA 3. When there is any problem in the results of this test, close examination B of the second stage is performed as described below.

Since sample DNA 3 used in the PCR-RFLP test of the first stage was digested into a long DNA fragment 16, a medium-length DNA fragment 17, and a short DNA fragment 18 in the test, it is impossible to read the nucleotide sequence of the DNA in close examination B of the second stage even when those DNA fragments are recovered. Then, the aliquot of the sample DNA stored at place (B) for close examination B of the second stage as shown in (c) is used for reading the nucleotide sequence by known methods (for example, see Sambrook J. et al. mentioned above). Thus, the presence of nucleotide sequence 15 corresponding to nucleotide sequence of interest 4 in sample DNA 3 is detected (g).

As described above, according to the embodiment of the present invention, it is possible to perform test A of the first stage simply and with low cost; and when there is any problem in the results, then it is possible to perform highly reliable close examination B of the second stage using the sample DNA stored in aliquots on the same device.

Either when sample DNA is recovered and recycled or when sample DNA is stored in aliquots for individual tests, the simple test of the first stage is not limited to those described in the embodiments of the present invention. Any technique capable of identifying one or a plurality of specific nucleotide sequences of DNA may be used.

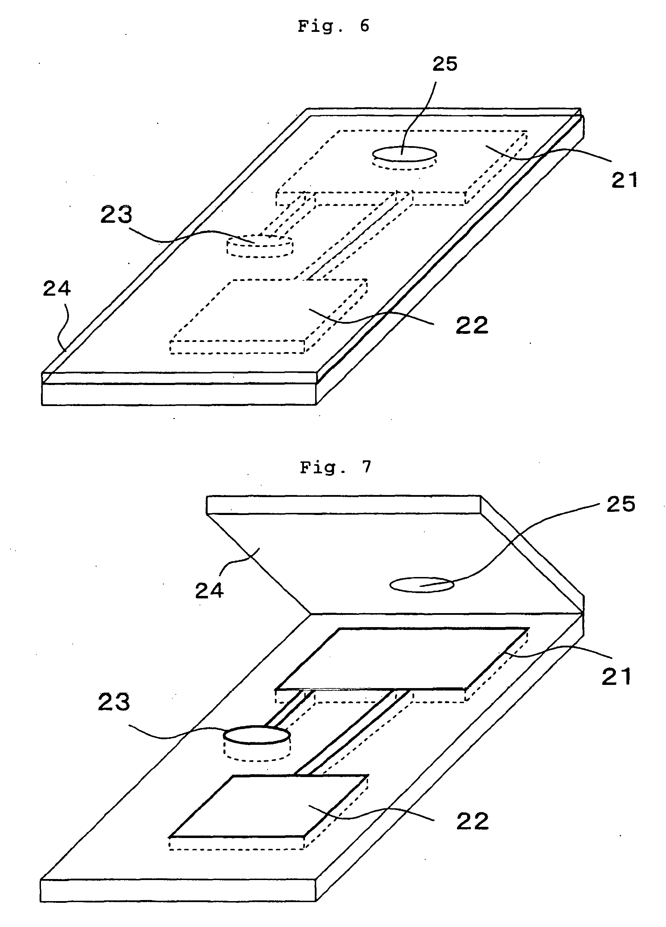

Fig. 6 illustrates a device which may be used when a simple test included in the two-step testing method as described above is performed using the microfluidics technique. As shown in this Figure, DNA extraction unit 21, detector unit 22 and DNA storage unit 23 are engraved in a flat board; and these units are connected with thin grooves. Lid 24, which is also a flat board, is placed upon this device. Lid 24 has hole 25 for feeding samples which would come above the DNA extraction unit. For a simple test, a sample (e.g., a piece of tuna meat) is fed through hole 25. DNA is extracted from the sample in DNA extraction unit 21 and then delivered to detector unit 22 through a thin groove, in which detection by a simple test is performed. When it is necessary to divide DNA into aliquots and store for a close examination of the second stage, an aliquot of the extracted DNA is also delivered to DNA storage unit 23 through another thin groove and stored therein, simultaneously with the delivery of another aliquot to detector unit 22. When a close examination is found necessary as a result of the simple test, this device as a whole may be delivered to the place of close examination. When a close examination is performed receiving the device as a whole, lid 24 is opened as shown in Fig. 7 and the DNA retained in DNA storage unit 23 and/or detector unit 22 is recovered and used in the close examination. By using such a device, confusion of samples, loss of DNA, mixing of contaminants, and so forth can be avoided; and it is possible to perform a simple test and a highly reliable close examination with an extremely small amount of sample.

### EXAMPLE 1

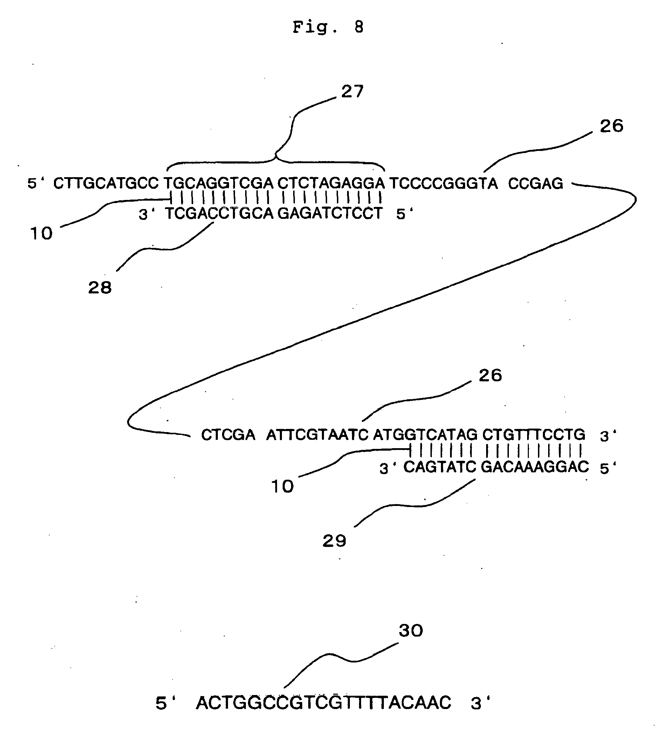

Whether or not nucleotide sequence of interest (i) 27 as shown in Fig. 8 is present in nucleotide sequence 26 of sample DNA was examined by the testing method of the invention. For DNA hybridization which is the simple test of the first stage, a fluorescently labeled probe was used which was prepared by labeling a probe DNA having probe nucleotide sequence 28 completely complementary to the nucleotide sequence of interest (i) 27 with a fluorescent dye Cy5, as shown in Fig. 8. For the reading of DNA nucleotide sequence which is the close examination of the second stage, a sequence primer was used which had sequence primer nucleotide sequence 29 completely complementary to a part of nucleotide sequence 26 of the sample DNA, as shown in Fig. 8.

According to the flow as shown in Fig. 4, first, 200 pmol of DNA having nucleotide sequence 26 of the sample DNA and biotinylated at its 5' end and 100 µg of streptavidin-coated magnetic beads (Bio-Adembeads Streptavidin, cat #0312, Ademtech) were added to 50 µl of TE buffer (10 mM Tris-HCl, 1 mM EDTA, pH 8.0), agitated and then left at room temperature for 30 minutes to thereby bind the 5' end of the DNA and the magnetic beads. With a magnetic micropipette PickPen (BIO-NOBILE), the magnetic beads in the solution were attracted and recovered. These beads were released and agitated in 50 µl of TE buffer placed in a separate tube to thereby wash the beads and the DNA bound thereto. This washing was repeated three times to remove unreacted DNA. Then, the contents of the tube were suspended in 50 µl of TE buffer placed in a separate tube to thereby prepare a suspension of bead-bound DNA (Fig. 4, (b)).

Subsequently, 100 pmol of DNA having probe nucleotide sequence 28 and labeled at its 5' end with fluorescent dye Cy5 in aqueous solution (2 µl) was added to 50 µl of the suspension of bead-bound DNA (Fig. 4, (c)). The resultant mixture was retained at 94°C for 30 seconds and then at 55°C for 5 minutes to thereby peform hybridization (Fig. 4, (d)).

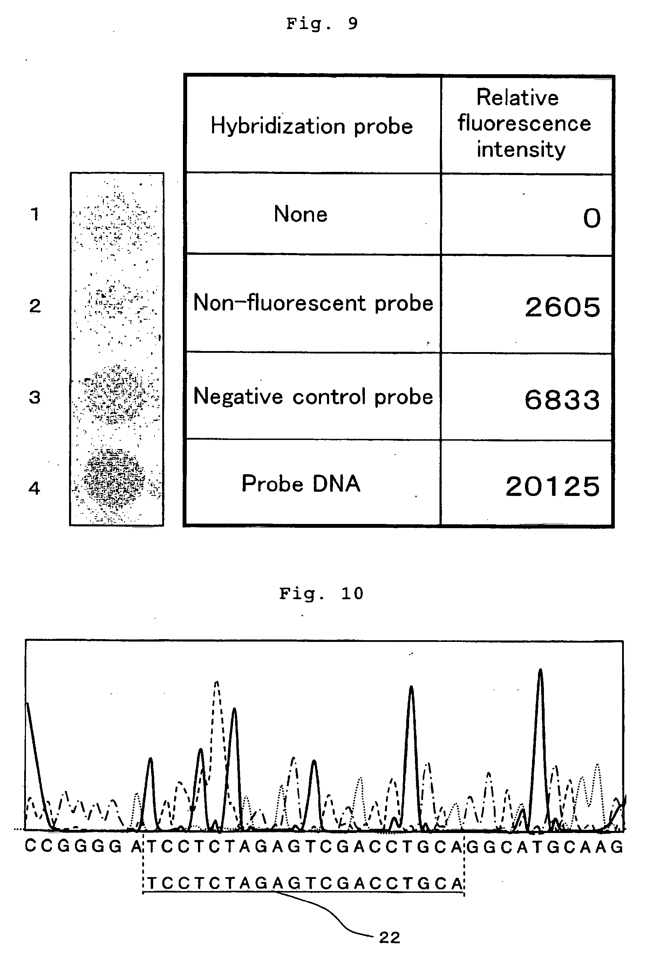

After the hybridization, magnetic beads in the reaction solution were recovered with a magnetic micropipette PickPen (e), and released and agitated in 50 µl of TE buffer placed in a separate tube to thereby wash the bead-bound DNA and the probe hybridizing thereto at room temperature. This washing was repeated three times to remove unreacted DNA. Then, the contents of the tube were suspended in 50 µl of TE buffer placed in a separate tube (Fig. 4, (f)). The fluorescence of this suspension was detected using FLA5000 fluoro-image analyzer (Fuji Film) under conditions of excitation wave length of 635 nm and use of R665 filter. Thus, results of detection of fluorescence by hybridization (i.e., the simple test of the first stage) were obtained. As shown in Fig. 9, the fluorescence intensity of this suspension was clearly greater than the fluorescence intensity of a suspension of bead-bound sample DNA 6 alone. Further, the fluorescence intensity of this suspension was clearly greater than the fluorescence intensity when similar hybridization was performed using a negative control probe which does not have a complementary nucleotide sequence to the sample DNA but has negative control probe nucleotide sequence 30 of the same length as that of the probe DNA, and is labeled with Cy5 at its 5' end. These results revealed that it is possible to detect the presence of nucleotide sequence of interest (i) 27 contained in nucleotide sequence 26 of the sample DNA by hybridization reaction using probe DNA 9 having probe nucleotide sequence 28.

Subsequently, the suspension was heated at 94°C for 2 minutes and then ice-cooled for 5 minutes to thereby dissociate the double strand (Fig. 4, (g)). Then, magnetic beads in the solution were recovered with a magnetic micropipette PickPen, and released and agitated in 50 µl of distilled water placed in a separate tube to thereby wash bead-bound sample DNA 6. This washing was repeated three times to remove dissociated probe DNA.

A sequencing reaction was performed with the recovered bead-bound sample DNA 6 and a sequence primer having sequence primer nucleotide sequence 29 using BigDye Terminator v1.1 Cycle Sequencing Kit (ABI) according to the Kit's protocol. Then, sample DNA 6 was sequenced using ABI PRISMR 310 Genetic Analyzer.

As a result, nucleotide sequence of interest (i) 27 was detected in a part of the resultant nucleotide sequence as shown in Fig. 10. Thus, it was demonstrated that the nucleotide sequence of interest can be detected by the technique in which sample DNA 3 used in the simple test is recovered and recycled to the close examination.

As stated above, according to the subject Example, it is possible to perform a simple and highly reliable testing by carrying out a simple test by hybridization to detect a specific nucleotide sequence, recovering the sample DNA used in the test, and reading the nucleotide sequence of the recovered sample DNA as a close examination.

### EXAMPLE 2

Whether or not it is possible to quantitatively detect the relative amount of nucleotide sequence of interest (ii) 31 present in nucleotide sequence 26 of sample DNA was examined by the testing method of the invention. Real time PCR was used as a simple test of the first stage, and the reading of the nucleotide sequence of DNA was used as a close examination of the second stage. Although real time PCR is a testing method that does not disrupt sample DNA, the sample DNA was not recovered after testing because an extremely small amount of sample DNA is sufficient for this method. The sample DNA was divided into two aliquots in advance, one of which was used in the real time PCR of the first stage and the other was used in the reading of nucleotide sequence in the second stage.

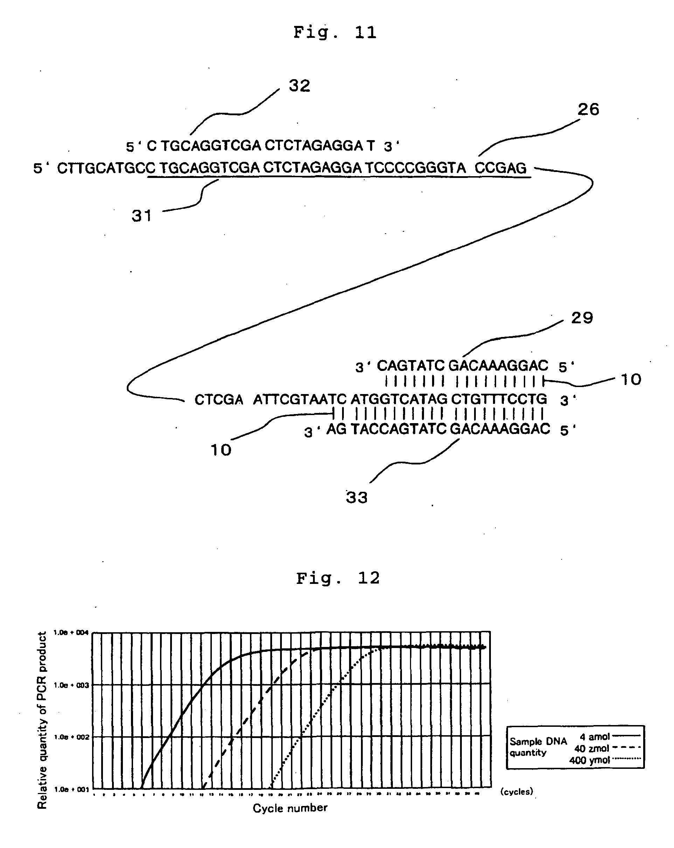

For the real time PCR, a pair of primers were used as shown in Fig. 11; one is RT primer [1] having nucleotide sequence 32 completely homologous to a 5' terminal region of nucleotide sequence of interest (ii) 31 and the other is RT primer [2] having nucleotide sequence 33 completely complementary to a 3' terminal region of nucleotide sequence of interest (ii) 31. For the reading of the nucleotide sequence of DNA (i.e., a close examination of the second stage), a sequence primer was used which has sequence primer nucleotide sequence 29 completely complementary to a 3' terminal region of nucleotide sequence 26 of the sample DNA and 5 bases shorter than nucleotide sequence 33 of RT primer [2], as shown in Fig. 11.

First, according to Fig. 5, a solution of DNA which has nucleotide sequence 26 of sample DNA and is biotinylated at its 5' end (concentration: 100 µM) was divided into two portions (Fig. 5, (b)) and poured into separate tubes (A) and (B) of 8-serial PCR tubes [Optical Tubes (8 Tubes/Strip), cat #4316567; ABI], sealed tightly with caps [ABI PRISM Optical Cap, 8 caps/strips(Flat), cat #4323032; ABI] and stored (Fig. 5, (c)).

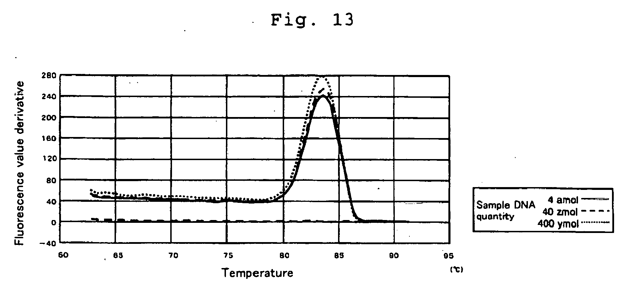

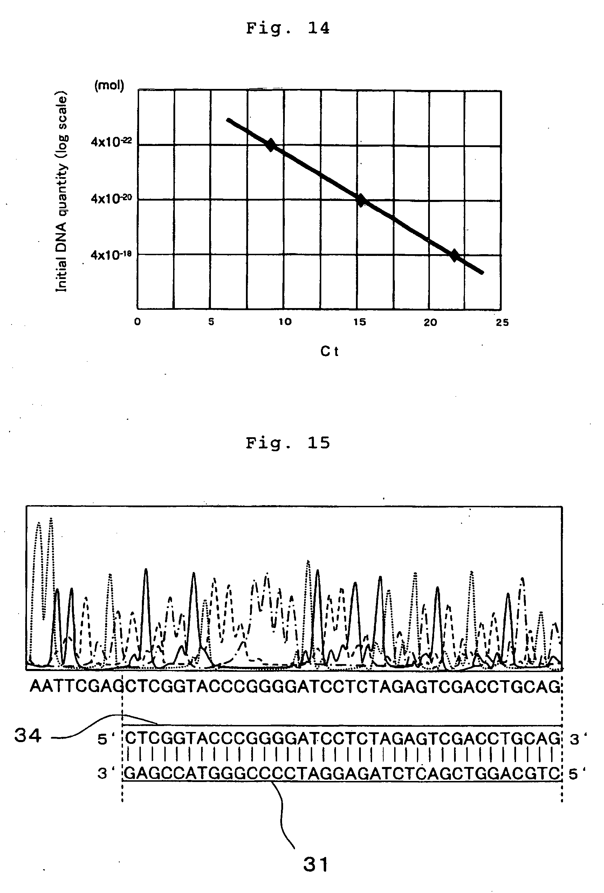

From tube (A), a 2 µl aliquot was taken three times and diluted with distilled water to prepare 2 pM solution, 20 fM solution and 0.2 fM solution. Then, 2 µl each of these solutions (containing 4 amol, 40 zmol and 400 ymol of sample DNA, respectively) was mixed with 5 µl of 10 x EX Taq buffer (attachment to Takara EX Taq, cat #RR001A; Takara Bio), 4 µl of 2.5 mM dNTP mix (attachment to Takara EX Taq, cat #RR001A; Takara Bio), 1 µl each of 10 µM RT primer [1] and 10 µM RT primer [2] (10 pmol each), 1.25 units of Taq polymerase (Takara ExTaq, cat #RR001A; Takara Bio) and 5 µl of 3,500 dilution of SYBR Green I (cat #F0512; Takara Bio) to prepare 50 µl of aqueous solution. Each of these aqueous solutions was added to a 96-well microtiter plate (Micro Amp Optical 96-well Reaction Plate, cat #N801-0560; ABI) and sealed tightly with caps (ABI PRISM OpticalCap, 8 caps/strips(Flat), cat #4323032; ABI). These samples were retained at 95°C for 2 minutes and then subjected to 40 cycles of the two steps of 95°C for 15 seconds and 60°C for 1 minute using ABI PRISM 7000 Sequence Detection System (ABI). Thus, PCR reaction was performed. During the PCR reaction, amplification of the DNA was monitored by measuring the fluorescence intensity of SYBR Green I. The results of measurement were summarized in graphs. As shown in Fig. 12, the higher the concentration of sample DNA was, the quantity of PCR product began to increase at a smaller cycle number and reached plateau at a smaller cycle number. In a negative control experiment using RT primers without complementarity to sample DNA, significant amplification of PCR product was not recognized. Therefore, it was confirmed that the amplification achieved with RT primers [1] and [2] was specific. Further, after completion of the PCR reaction, the solution was retained at 95°C for 15 seconds and at 60°C for 1 minute and then heated to 95°C over 20 minutes to perform melting curve analysis. As a result, it was demonstrated from the negative primary differential curves of relative fluorescence value shown in Fig. 13 that all the amplified DNAs were one sequence having the same Tm value. In a negative control experiment using RT primers without complementarity to sample DNA, the melting curve became a strait line at the base level without temperature dependency, and no PCR product was observed. Thus, again it was confirmed that the amplification achieved with RT primers [1] and [2] was specific. Further, in the calibration curve prepared based on Fig. 12, logarithmic values of the quantity of sample DNA and Ct values exhibited a linear relationship as shown in Fig. 14. Therefore, it was confirmed that nucleotide sequence of interest (ii) 26 was contained in the sample, and it was also shown that the amount thereof can be examined quantitatively using the technique of real time PCR. Needless to say, qualitative examination can also be performed using the technique of real time PCR.

Subsequently, a sequencing reaction was performed with 2 pmol of sample DNA having nucleotide sequence 26 dispensed into tube (B) in advance and a sequence primer having sequence primer nucleotide sequence 29 using BigDye Terminator v1.1 Cycle Sequencing Kit (cat #4337449; ABI) according to the Kit's protocol. Then, sample DNA was sequenced using ABI PRISMR 310 Genetic Analyzer.

As a result, as shown in Fig. 15, nucleotide sequence 34 which is completely complementary to nucleotide sequence of interest (i) 31 was detected in a part of the resultant nucleotide sequence. Thus, it was demonstrated that the nucleotide sequence of interest can be detected.

When there are a plurality of nucleotide sequences of interest, the presence or absence of the nucleotide sequences of interest is judged by performing real time PCR reactions on these sequences simultaneously in one reaction vessel and examining whether or not amplification of DNA is detected. When amplification of any of the nucleotide sequences of interest has been detected, a close examination of the second stage is performed and the type of the nucleotide sequence of interest present in the sample is identified. In this case, the simple test only detects the presence or absence of nucleotide sequences of interest. Therefore, the fluorescence dye used in a plurality of real time PCR reactions may be either single or plural.

### BRIEF DESCRIPTION OF THE DRAWINGS

Fig. 1 illustrates a flow of the two-step DNA testing method where a simple test of the first stage does not disrupt sample DNA.
Fig. 2 illustrates a flow of the two-step DNA testing method where a simple test of the first stage disrupts sample DNA.
Fig. 3 shows a flow of the preparation of sample DNA from an organism to be tested.
Fig. 4 illustrates a flow of the two-step testing where sample DNA used in a simple test of the first stage is recovered and recycled to a close examination of the second stage.
Fig. 5 illustrates a flow of the two-step testing where sample DNA is divided into two aliquots and stored on the same device, and one aliquot is used in a simple test of the first stage and the other is used in a close examination of the second stage.
Fig. 6 shows a testing device using the microfluidics technique.
Fig. 7 shows the testing device of Fig. 6 with its lid opened.
Fig. 8 is a diagram showing the nucleotide sequences of individual DNAs used in the testing of Example 1 and the complementarity thereof.
Fig. 9 shows the results of a simple test of the first stage in Example 1.
Fig. 10 shows the results of a close examination of the second stage in Example 1.
Fig. 11 is a diagram showing the nucleotide sequences of individual DNAs used in the testing of Example 2 and the complementarity thereof.
Fig. 12 is a graph showing proliferation curves of the results of a simple test of the first stage in Example 2.
Fig. 13 is a graph showing that amplification of DNA was successful in a simple test of the first stage in Example 2.
Fig. 14 shows the calibration curve prepared from the results of a simple test of the first stage in Example 2.
Fig. 15 is a diagram showing the results of a close examination of the second stage in Example 2.

### Figure Legends:

1. Group of organisms to be tested
2. Nucleic acid from an organism to be tested
3. Sample DNA
4. Nucleotide sequence of interest
5. Magnetic beads
6. Sample DNA bound to a bead
7. Group of sample DNAs bound to beads
8. Fluorescent dye
9. Probe DNA
10. Fluorescence-labeled probe
11. Group of fluorescence-labeled probes
12. Group of unreacted fluorescence-labeled probes
13. Double-strand formation
14. Magnetic pipette
15. DNA nucleotide sequence
16. Digested long DNA fragment
17. Digested medium-length DNA fragment
18. Digested short DNA fragment
19. Electrophoresis gel
20. Electrophoresis apparatus
21. DNA extraction unit
22. Detector unit
23. DNA storage unit
24. Lid
25. Hole for sample feeding
26. Nucleotide sequence of sample DNA
27. Nucleotide sequence of interest (i)
28. Nucleotide sequence of probe
29. Nucleotide sequence of sequence primer
30. Nucleotide sequence of negative control primer
31. Nucleotide sequence of interest (ii)
32. Nucleotide sequence of RT primer [1]
33. Nucleotide sequence of RT primer [2]
34. Nucleotide sequence of complementary strand to sequence of interest (ii)

The present specification encompasses the contents disclosed in the specification and/or the drawings of Japanese Patent Application No. 2006-072581 based on which the present application claims priority.

## Claims

1. A method of testing whether a sequence of interest is contained in a sample nucleic acid or not, comprising
performing a simple test on the sample nucleic acid, wherein the simple test is a quantitative test on the sample nucleic acid, and
subjecting the same sample nucleic acid to a close examination when it has been judged in said simple test that the sequence of interest is contained therein in more than a specific amount, wherein the close examination is an examination performed by reading the nucleotide sequence of the sample nucleic acid,
wherein said method is carried out using a device in which
(a) a DNA extraction unit (21), detector unit (22) and DNA storage unit (23) are engraved on a flat board; and
(b) said DNA extraction unit (21) is connected to said detector unit (22) by a thin groove and said DNA extraction unit (21) is connected to said DNA storage unit (23) by a thin groove; and
(c) a lid (24), which is a flat board, is placed on the device, wherein said lid has a hole (25) located above the DNA extraction unit (21) through which samples can be fed into said DNA extraction unit;
wherein said method comprises
feeding a sample through the hole (25) into the DNA extraction unit (21), extracting DNA from the sample in DNA extraction unit (21),
dividing the extracted DNA in said DNA extraction unit (21) into two aliquots,
delivering the first aliquot to the detector unit (22) and simultaneously delivering the second aliquot to the DNA storage unit (23), and
carrying out the simple test on said first aliquot in the detector unit (22)
wherein the two aliquots are both stored on the same device for performing the simple test and the close examination.

2. The method according to claim 1, wherein the sequence of interest is a sequence occurring specifically in a species of an organism.

3. The method according to claim 1, wherein the sequence of interest is a sequence occurring specifically in a genetically modified organism.

4. The method according to claim 1, wherein a plurality of sequences of interest can be tested simultaneously in one simple test.

5. The method according to, any one of the preceding claims wherein said close examination comprises opening said lid (24), recovering the DNA retained in the DNA storage unit (23) and/or the detector unit (22) and carrying out a close examination on said DNA.

## Patentansprüche

1. Verfahren zur Untersuchung, ob eine Sequenz von Interesse in einer Probennucleinsäure enthalten ist oder nicht, umfassend
Durchführen eines einfachen Tests an einer Probennucleinsäure, wobei der einfache Test ein quantitativer Test an der Probennucleinsäure ist, und
Unterwerfen derselben Probennucleinsäure einer genauen Untersuchung, wenn in dem einfachen Test beurteilt wurde, dass die Sequenz von Interesse darin in mehr als einer spezifischen Menge enthalten ist, wobei die genaue Untersuchung eine Untersuchung ist, die durch Lesen der Nucleotidsequenz der Probennucleinsäure durchgeführt wird,
wobei das Verfahren unter Verwendung einer Vorrichtung durchgeführt wird, in der
(a) eine DNA-Extraktionseinheit (21), eine Detektoreinheit (22) und eine DNA-Speichereinheit (23) auf einer flachen Platte eingraviert sind und
(b) die DNA-Extraktionseinheit (21) mit der Detektoreinheit (22) durch eine dünne Rille verbunden ist, und die DNA-Extraktionseinheit (21) mit der DNA-Speichereinheit (23) mit einer dünnen Rille verbunden ist, und
(c) ein Deckel (24), der eine flache Platte ist, auf die Vorrichtung gelegt ist, wobei der Deckel ein Loch (25) hat, das über der DNA-Extraktionseinheit (21) lokalisiert ist, durch das Proben in die DNA-Extraktionseinheit eingeführt werden können;
wobei das Verfahren umfasst
Einführen einer Probe durch das Loch (25) in die DNA-Extraktionseinheit (21),
Extrahieren der DNA aus der Probe in der DNA-Extraktionseinheit (21),
Aufteilen der extrahierten DNA in der DNA-Extraktionseinheit (21) in zwei Aliquots,
Abgeben des ersten Aliquots an die Detektoreinheit (22) und gleichzeitig Abgeben des zweiten Aliquots an die DNA-Speichereinheit (23) und
Durchführen des einfachen Tests an dem ersten Aliquot in der Detektoreinheit (22),
wobei die zwei Aliquots beide an der selben Vorrichtung zur Durchführung des einfachen Tests und der genauen Untersuchung gespeichert werden.

2. Verfahren gemäß Anspruch 1, wobei die Sequenz von Interesse eine Sequenz ist, die spezifisch in einer Spezies eines Organismus vorkommt.

3. Verfahren gemäß Anspruch 1, wobei die Sequenz von Interesse eine Sequenz ist, die spezifisch in einem genetisch modifizierten Organismus vorkommt.

4. Verfahren gemäß Anspruch 1, wobei eine Vielzahl von Sequenzen von Interesse gleichzeitig in einem einfachen Test getestet werden kann.

5. Verfahren gemäß einem der vorangehenden Ansprüche, wobei die genaue Untersuchung Öffnen des Deckels (24), Gewinnen der DNA, die in der DNA-Speichereinheit (23) und/oder der Detektoreinheit (22) zurückgehalten wird, und Durchführen einer genauen Untersuchung an der DNA umfasst.

## Revendications

1. Procédé de test permettant d'établir si, oui ou non, une séquence intéressante est contenue dans un acide nucléique d'un échantillon, lequel procédé comporte les étapes suivantes :
- réaliser un simple test sur l'acide nucléique d'échantillon, lequel simple test est un test quantitatif auquel on soumet l'acide nucléique d'échantillon,
- et soumettre le même acide nucléique d'échantillon à un examen approfondi si l'on estime, suite audit simple test, que la séquence intéressante y est contenue en une quantité supérieure à un certain seuil spécifié, lequel examen approfondi est un examen réalisé par lecture de la séquence de nucléotides de l'acide nucléique d'échantillon;
lequel procédé est mis en oeuvre au moyen d'un dispositif dans lequel
a) une unité d'extraction d'ADN (21), une unité détecteur (22) et une unité réserve d'ADN (23) sont gravées sur un panneau plat,
b) ladite unité d'extraction d'ADN (21) est reliée à ladite unité détecteur (22) par une rainure mince et ladite unité d'extraction d'ADN (21) est reliée à ladite unité réserve d'ADN (23) par une rainure mince,
c) et un couvercle (24), constitué d'un panneau plat, est placé sur le dispositif, lequel couvercle est percé d'un trou (25), situé au-dessus de l'unité d'extraction d'ADN (21), par lequel on peut introduire des échantillons dans ladite unité d'extraction d'ADN ;
et lequel procédé comporte les opérations suivantes :
- introduire un échantillon, par le trou (25), dans l'unité d'extraction d'ADN (21),
- extraire de l'ADN de l'échantillon, dans l'unité d'extraction d'ADN (21),
- diviser en deux fractions aliquotes cet ADN extrait dans l'unité d'extraction d'ADN (21),
- envoyer la première fraction aliquote dans l'unité détecteur (22) et envoyer en même temps la deuxième fraction aliquote dans l'unité réserve d'ADN (23),
- et réaliser le simple test sur ladite première fraction aliquote dans l'unité détecteur (22),
étant entendu que l'on garde les deux fractions aliquotes sur le même dispositif pour effectuer le simple test et l'examen approfondi.

2. Procédé conforme à la revendication 1, dans lequel la séquence intéressante est une séquence qui se trouve spécifiquement chez une espèce d'un organisme.

3. Procédé conforme à la revendication 1, dans lequel la séquence intéressante est une séquence qui se trouve spécifiquement chez un organisme génétiquement modifié.

4. Procédé conforme à la revendication 1, dans lequel on peut tester simultanément, en un seul simple test, la présence de plusieurs séquences intéressantes.

5. Procédé conforme à l'une des revendications précédentes, dans lequel ledit examen approfondi comprend les opérations suivantes :
- ouvrir ledit couvercle (24),
- récupérer l'ADN conservé dans l'unité réserve d'ADN (23) et/ou dans l'unité détecteur (22),
- et soumettre cet ADN à un examen approfondi.
